# EUROPÄISCHE PATENTSCHRIFT

(11) **EP 0 795 540 B1**
(45) Veröffentlichungstag und Bekanntmachung des Hinweises auf die Patenterteilung: **29.09.1999**
(21) Anmeldenummer: 97103931.8
(22) Anmeldetag: 10.03.1997
(51) Int. Cl.: C07C 231/02, C07C 237/22

(54) **Verfahren zur Herstellung von N-Lauroyl-L-glutaminsäure-di-n-butylamid**
Process for the preparation of N-Lauroyl-L-glutamic acid di-n-butylamide
Procédé pour la préparation du N-Lauroyl-L-glutamique acide-di-n-butylamide

(30) Priorität: 15.03.1996 DE 19610323
(43) Veröffentlichungstag der Anmeldung: 17.09.1997
(73) Patentinhaber: Clariant GmbH, 65929 Frankfurt am Main (DE)
(72) Erfinder: Engelhardt, Fritz, Dr., Chesapeake, Virginia 23320 (US); Müller, Manfred, 63571 Gelnhausen (DE); Wessling, Michael, Dr., Charlotte, North Carolina 28210 (US)

(56) Entgegenhaltungen:
- PATENT ABSTRACTS OF JAPAN vol. 010, no. 139 (C-348), 22.Mai 1986 & JP 61 000050 A (AJINOMOTO KK), 6.Januar 1986,

## Beschreibung

Die vorliegende Anmeldung betrifft ein Verfahren zur Herstellung von N-Lauroyl-L-glutaminsäure-di-n-butylamid durch Umsetzung von N-Lauroyl-L-glutaminsäuredimethylester mit n-Butylamin in Gegenwart eines Kohlenwasserstoffs oder Kohlenwasserstoffgemischs als Hilfslösungsmittel.

N-Lauroyl-L-glutaminsäure-di-n-butylamid ist in der Patentliteratur vielfältig beschrieben als Verdickungsmittel für meist hydrophobe, unpolare Medien. So wird die Verbindung als ausgezeichneter Verdicker zur Herstellung von druckempfindlichem Papier, von photosensitiven Druckplatten, von auf Butan basierendem "Fest-Brennstoff' und von Vulkanisaten, zur Eindickung von photographischen Entwickler-Abfall-Lösungen und für verschiedene kosmetische Anwendungen, beispielsweise Lippenstifte, beschrieben.
Die für diese Anwendungen als sehr nützlich eingestuften Eigenschaften des Produktes stellen sich allerdings bei dessen Herstellung als äußerst hinderlich heraus. Dies gilt insbesondere für eine unter ökologisch-ökonomischen Gesichtspunkten akzeptable Verfahrensführung, die nach Möglichkeit bei guten Ausbeuten zu einem Produkt hoher Reinheit führen sollte, ohne größere Mengen an Abfall und Abwasser zu produzieren.
Für die Darstellung von Carbonsäureamiden und Dicarbonsäurediamiden gibt es eine Vielzahl von in der Literatur hinlänglich zitierten Syntheserouten. Man kann dabei je nach Reaktivität und vorliegendem Einzelfall von den unterschiedlichsten Carbonsäure-Derivaten ausgehen, wie beispielsweise von Carbonsäurechloriden, - anhydriden, -estern oder auch anderen Carbonsäureamiden. Die im Hinblick auf Umsatz und Ausbeute in der Regel vielversprechendste Variante über das Säurechlorid enffällt im vorliegenden Fall, da N-Lauroyl-L-glutaminsäuredichlorid nur schwer zugänglich ist. Die Direktkondensation von Carbonsäuren mit Aminen erfordert in der Regel drastischere Bedingungen, wie zum Beispiel höhere Temperatur und das Arbeiten unter erhöhtem Druck. Die Darstellung von N-Lauroyl-L-glutaminsäure-di-n-butylamid durch Direktkondensation ist in CA 105(17), 153544s (1986) (entspricht JP 61 00050 A2) beschrieben. Eigene Versuche der Anmelderin ergaben hier allerdings lediglich Ausbeuten zwischen 40 und 60%, wobei das Produkt in der Regel noch größere Mengen an Verunreinigungen enthielt.
Bei der Umsetzung von Carbonsäureestern mit Aminen wird üblicherweise in einem inerten Lösungsmittel oder in dem Alkohol des Esters als Lösungsmittel gearbeitet. Normalerweise wird dabei ein Aminüberschuß verwendet, um den Umsatz möglichst vollständig gestalten zu können. Sofern das gewünschte Produkt nicht leicht kristallisiert und umkristallisiert werden kann, erfolgt dessen Isolierung über eine wäßrige Aufarbeitung, wobei in der Regel große Mengen an Amin- und Alkoholhaltigen Abwässern anfallen. Erfahrungsgemäß zieht dies umfangreiche Aufarbeitungsschritte nach sich.
Die Umsetzung von N-Lauroyl-L-glutaminsäuredimethylester mit n-Butylamin gestaltet sich äußerst schwierig, wobei insbesondere die Aufarbeitung nach der eigentlichen Umsetzung problembeladen ist. N-Lauroyl-L-glutaminsäure-di-n-butylamid neigt nämlich in sehr vielen inerten Lösungsmitteln wie beispielsweise Aromaten wie Toluol, aber auch in Glykolen, Ethern usw. zur Gelbildung und zum Einschluß von Verunreinigungen, insbesondere von n-Butylamin, das ja wie erwähnt im Überschuß eingesetzt werden muß. Die genannten Lösungsmittel können daher nicht verwendet werden. Geeignete Lösungsmittel zur Umkristallisation des Produktes zwecks Reinigung sind wegen der genannten Eigenschaften praktisch nicht auffindbar. Wählt man daher nach erfolgter Umsetzung eine wäßrige Aufarbeitung, so erhält man ein weitgehend Butylamin-freies Produkt von akzeptabler Reinheit und Ausbeute nur unter großem Aufwand und unter Anfall von großen Abwassermengen, die neben verschiedenen organischen Verunreinigungen den bei der Synthese eingesetzten Butylaminüberschuß, sowie das bei der Amidbildung freigesetzte Methanol enthalten.

Es besteht somit ein dringender Bedarf nach einem Syntheseweg für N-Lauroyl-L-glutaminsäure-di-n-butylamid, der die oben geschilderten Nachteile nicht aufweist. Überraschenderweise wurde nun gefunden, daß ausgehend von N-Lauroyl-L-glutaminsäuredimethylester ein sehr reines, praktisch Butylamin-freies N-Lauroyl-L-glutaminsäure-di-n-butylamid in guten Ausbeuten und ohne jeglichen Abwasseranfall zugänglich ist, wenn man die Reaktion in Gegenwart eines Kohlenwasserstoffes oder eines Kohlenwasserstoffgemisches als Hilfslösungsmittel ausführt. Auf diesem Wege ist eine einfache Umsetzung des Diesters zum Diamid bei reaktionsfördender, teilweiser Entfernung des freigesetzten Methanols, sowie eine problemlose Aufarbeitung und Isolierung des Produktes möglich.

Die vorliegende Erfindung betrifft somit ein Verfahren zur Herstellung von N-Lauroyl-L-glutaminsäure-di-n-butylamid durch Umsetzung von N-Lauroyl-L-glutaminsäuredimethylester mit n-Butylamin, dadurch gekennzeichnet, daß die Umsetzung in Gegenwart eines Kohlenwasserstoffs oder eines Kohlenwasserstoffgemisches als Hilfslösungsmittel ausgeführt wird.

Bezogen auf N-Lauroyl-L-glutaminsäuredimethylester wird n-Butylamin sinnvollerweise im Überschuß eingesetzt. Bevorzugt werden pro Mol Ester 2,1 bis 10 Mol Amin, besonders bevorzugt pro Mol Ester 3 bis 6 Mol Amin, eingesetzt. Das n-Butylamin fungiert auf diese Weise gleichzeitig als Lösungsmittel.

Die als Hilfslösungsmittel eingesetzten Kohlenwasserstoffe oder Kohlenwasserstoffgemische haben bevorzugt Siedepunkte von über 60°C, besonders bevorzugt von über 100°C. Beispiele für geeignete Kohlenwasserstoffe sind n-Nonan, n-Decan, n-Undecan, n-Dodecan, sowie mehrfach alkylierte Cyclohexanderivate mit Siedepunkten von über 100°C, wie beispielsweise Trimethylcyclohexan, Methylethylcyclohexan und Diethylcyclohexan.
Geeignete Kohlenwasserstoffgemische sind insbesondere Mischungen aus den oben genannten Kohlenwasserstoffen, insbesondere hochsiedender Cyclohexanderivate. Solche Mischungen sind dem Fachmann bekannt und handelsüblich. Bezogen auf N-Lauroyl-L-glutaminsäuredimethylester werden die Kohlenwasserstoffe oder Kohlenwasserstoffgemische bevorzugt in Mengen von 20 bis 300 Gew.%, besonders bevorzugt in Mengen von 50 bis 150 Gew.%, eingesetzt.

Nach erfolgter Umsetzung der Reaktanten erfolgt die Aufarbeitung und Isolierung des Produktes. In einer bevorzugten Ausführungsform des erfindungsgemäßen Verfahrens geschieht dies derart, daß zunächst überschüssiges n-Butylamin destillativ entfernt und anschließend das Produkt durch Zugabe eines polaren Lösungsmittels gefällt wird.
Geeignete polare Lösungsmittel sind insbesondere Alkohole, Ether, Ester und Alkanone, beispielsweise Aceton.
Wird für die Umsetzung ein Kohlenwasserstoff oder ein Kohlenwasserstoffgemisch mit ausreichend hohem Siedepunkt, insbesondere von über 100°C, gewählt, so kann das n-Butylamin (Siedepunkt 77,8°C) auf diese Weise über eine Kolonne zurückgewonnen und gegebenenfalls wiederverwendet werden.
Auch nach Entfernen des n-Butylamins bleibt das Reaktionsgemisch handhabbar und durchläuft keine zu hohe Viskosität.
Zur Fällung des Produkts wird vorteilhafterweise eine solche Menge des polaren Lösungsmittels verwendet, daß es vollständig und in filtrierbarer Form ausfällt. Nach der Fällung wird das Produkt abfiltriert und kann gewünschtenfalls mit weiterem Lösemittel gewaschen werden. Durch diese Nachwäsche können die an sich schon geringen Anteile an Verunreinigungen weiter herabgesetzt werden. In der Regel liegt die Reinheit des nach dem erfindungsgemäßen Verfahren erhaltenen Produktes bei >95%, typischerweise sogar bei >97%.

Der als Ausgangsprodukt benötigte N-Lauroyl-L-glutaminsäuredimethylester ist eine literaturbekannte Verbindung und kann nach dem Fachmann bekannten Verfahren hergestellt werden.

### Beispiel 1

In einem 1-l-Quickfit-Kolben mit glasummanteltem Thermofühler, Gaseinleitung, Edelstahl-Ankerrührer und beheizbarer (67-70°C) Vorkolonne mit aufgesetzter Claisen-Brücke und nachgeschalteter Kühlfalle werden unter leichtem N₂-Strom 110g N-Lauroyl-L-glutaminsäuredimethylester (LGA-DME) und 100g eines Gemisches aus Cyclohexan-Derivaten (Trimethylcyclohexan, Methyethylcyclohexan, Diethylcyclohexan im ungefähren Verhältnis von 45 : 25 : 10) vorgelegt. Man dosiert innerhalb 30 min 110g n-Butylamin ein und erhitzt zum Rückfluß. Während der Umsetzung werden über die temperierte Vorkolonne Anteile des bei der Amidierung freigesetzten Methanols mit wenig n-Butylamin abgetrennt. Nach ca. 8 h Rückfluß zeigt DC-Kontrolle quantitativen Umsatz an. Man destilliert zunächst bei Normaldruck, dann unter Vakuum den Überschuß an n-Butylamin ab und fällt das Produkt unter Zusatz von Aceton aus. Der entstehende Niederschlag wird abgesaugt, mit Aceton gewaschen, getrocknet und zerkleinert. Man erhält das Zielprodukt N-Lauroyl-L-glutaminsäure-di-n-butylamid in guter Ausbeute von etwa 80% und in ausgezeichneter Reinheit (LGB 99%(HPLC), nBuNH₂ 50ppm (GC), andere Verunreinigungen < 1%) in Form eines farblosen Pulvers vom Schmelzpunkt 150 -152°C.

### Vergleichsbeispiel

In einem 1-l-Quickfit-Kolben mit glasummanteltem Thermofühler, Gaseinleitung, Edelstahl-Ankerrührer und Rückflußkühler werden unter leichtem N₂-Strom 110g N-Lauroyl-L-glutaminsäuredimethylester (LGA-DME) in 20g MeOH vorgelegt. Man dosiert innerhalb von 30 min 110g N-Butylamin ein und erhitzt 8 h zum Rückfluß. Man löst das Reaktionsgemisch in 100g Methanol auf und dosiert 400g Wasser in die Lösung, wobei das Produkt gelartig ausfällt. Um die u.a. Werte für Rest-Butylamin zu erreichen, wird wie folgt aufgearbeitet:
Der erhaltene teigige Niederschlag wird abgesaugt, in 300g 1 N HCl eingerührt, mit 2 x 250g Wasser bis pH 3 gewaschen, 2 x in 300 g 1 N NaOH eingerührt und schließlich mit ca. 2000g Wasser solange gewaschen, bis das Filtrat neutral reagiert. Die entstehende teigige Paste wird getrocknet und zerkleinert. Man erhält das Zielprodukt N-Lauroyl-L-glutaminsäure-di-n-butylamid in einer Ausbeute von etwa 70% und in mäßiger Reinheit (LGB 78% (HPLC), n-BuNH₂ 180ppm (GC), Laurinsäurebutylamid 5% (HPLC), andere Verunreinigungen > 15%) in Form eines weitgehend farblosen Pulvers vom Schmelzbereich 135-145°C. Die großen Mengen an anfallendem Abwasser müssen aufgearbeitet werden.

## Patentansprüche

1. Verfahren zur Herstellung von N-Lauroyl-L-glutaminsäure-di-n-butylamid durch Umsetzung von N-Lauroyl-L-glutaminsäuredimethylester mit n-Butylamin, dadurch gekennzeichnet, daß die Umsetzung in Gegenwart eines Kohlenwasserstoffs oder eines Kohlenwasserstoffgemisches als Hilfslösungsmittel ausgeführt wird.

2. Verfahren gemäß Anspruch 1, dadurch gekennzeichnet, daß pro Mol N-Lauroyl-L-glutaminsäuredimethylester 3 bis 6 Mol n-Butylamin eingesetzt werden.

3. Verfahren gemäß Anspruch 1 und/oder 2, dadurch gekennzeichnet, daß Kohlenwasserstoffe oder Kohlenwasserstoffgemische mit einem Siedepunkt von über 100°C eingesetzt werden.

4. Verfahren gemäß einem oder mehreren der Ansprüche 1 bis 3, dadurch gekennzeichnet, daß als Kohlenwasserstoffe oder Kohlenwasserstoffgemische n-Nonan, n-Decan, n-Undecan, n-Dodecan oder mehrfach alkylierte Cylohexanderivate mit Siedepunkten von über 100°C eingesetzt werden.

5. Verfahren gemäß einem oder mehreren der Ansprüche 1 bis 4, dadurch gekennzeichnet, daß die Kohlenwasserstoffe oder Kohlenwasserstoffgemische in Mengen von 50 bis 150 Gew.%, bezogen auf N-Lauroyl-L-glutaminsäuredimethylester, eingesetzt werden.

6. Verfahren gemäß einem oder mehreren der Ansprüche 1 bis 5, dadurch gekennzeichnet, daß nach erfolgter Umsetzung überschüssiges n-Butylamin destillativ entfernt und anschließend N-Lauroyl-L-glutaminsäure-di-n-butylamid durch Zugabe eines polaren Lösungsmittels oder Lösungsmittelgemisches gefällt wird.

7. Verfahren gemäß Anspruch 6, dadurch gekennzeichnet, daß zur Fällung des N-Lauroyl-L-glutaminsäure-di-n-butylamids Aceton eingesetzt wird.

## Claims

1. A process for the preparation of N-lauroyl-L-glutamic acid di-n-butylamide by reaction of N-lauroyl-L-glutamic acid dimethyl ester with n-butylamine, which comprises carrying out the reaction in the presence of a hydrocarbon or a hydrocarbon mixture as an auxiliary solvent.

2. The process as claimed in claim 1, wherein 3 to 6 mol of n-butylamine are employed per mole of N-lauroyl-L-glutamic acid dimethyl ester.

3. The process as claimed in claim 1 and/or 2, wherein a hydrocarbon or hydrocarbon mixture having a boiling point above 100°C is employed.

4. The process as claimed in one or more of claims 1 to 3, wherein n-nonane, n-decane, n-undecane, n-dodecane or a polyalkylated cyclohexane derivative having a boiling point above 100°C is employed as the hydrocarbon or hydrocarbon mixture.

5. The process as claimed in one or more of claims 1 to 4, wherein the hydrocarbon or hydrocarbon mixture is employed in amounts of 50 to 150% by weight, based on the N-lauroyl-L-glutamic acid dimethyl ester.

6. The process as claimed in one or more of claims 1 to 5, wherein, after the reaction has taken place, excess n-butylamine is removed by distillation and N-lauroyl-L-glutamic acid di-n-butylamide is then precipitated by addition of a polar solvent or solvent mixture.

7. The process as claimed in claim 6, wherein acetone is employed for the precipitation of the N-lauroyl-L-glutamic acid di-n-butylamide.

## Revendications

1. Procédé pour la préparation de N-lauroyl-L-acide glutamique-di-n-butyl-amide par réaction de N-lauroyl-L-glutamate de diméthyle avec la n-butylamine, caractérisé en ce que la réaction est conduite en présence d'un hydrocarbure ou d'un mélange d'hydrocarbures comme co-solvant.

2. Procédé selon la revendication 1, caractérisé en ce qu'on utilise par mole de N-lauroyl-L-glutamate de diméthyle 3 à 6 moles de n-butylamine.

3. Procédé selon la revendication 1 et/ou 2, caractérisé en ce qu'on utilise des hydrocarbures ou des mélanges d'hydrocarbures avec un point d'ébullition de plus de 100°C.

4. Procédé selon une ou plusieurs des revendication 1 à 3, caractérisé en ce qu'on utilise comme hydrocarbures ou mélanges d'hydrocarbures le n-nonane, le n-décane, le n-undécane, le n-dodécane ou des dérivés plusieurs fois alkylés du cyclohexane ayant des points d'ébullition de plus de 100°C.

5. Procédé selon une ou plusieurs des revendication 1 à 4, caractérisé en ce qu'on utilise les hydrocarbures ou mélanges d'hydrocarbures en quantités de 50 à 150% en poids, par rapport au N-lauroyl-L-glutamate de diméthyle.

6. Procédé selon une ou plusieurs des revendication 1 à 5, caractérisé en ce qu'on élimine, après avoir effectué la réaction, le n-butylamine en excès par distillation et en ce qu'on fait ensuite précipiter le N-lauroyl-L-acide glutamique-di-n-butyl-amide par addition d'un solvant ou d'un mélange de solvants polaire.

7. Procédé selon la revendication 6, caractérisé en ce qu'on utilise l'acétone pour la précipitation du N-lauroyl-L-acide glutamique-di-n-butylamide.
